# EUROPÄISCHE PATENTANMELDUNG

(11) **EP 4 782 860 A1**
(43) Veröffentlichungstag der Anmeldung: **29.07.2026**
(21) Anmeldenummer: 25154397.1
(22) Anmeldetag: 28.01.2025
(51) Int. Cl.: G01R 33/24, G01R 33/483, G01R 33/54, G01R 33/561, G01R 33/565, G01R 33/28

(54) **MAGNETRESONANZSEQUENZ MIT FREIEM ANREGUNGSPULS UND FORMGEBUNDENEN REFOKUSSIERUNGSPULSEN**

(71) Anmelder: Siemens Healthineers AG, 91301 Forchheim (DE)
(72) Erfinder: Herrler, Jürgen, 91058 Erlangen (DE)
(74) Vertreter: Siemens Healthineers Patent Attorneys

(57) **Zusammenfassung**

Die Erfindung betrifft ein Verfahren zum Bereitstellen einer Magnetresonanzsequenz, eine Magnetresonanzvorrichtung und ein Computerprogrammprodukt. Bei dem Verfahren wird ein Magnetfeldverteilungsdatensatz bereitgestellt. Zudem wird ein Anregungspuls ermittelt, der eine Anregungspulsform aufweist, wobei die Anregungspulsform in Abhängigkeit von dem Magnetfeldverteilungsdatensatz ermittelt wird. Ferner wird eine Refokussierungspulsform vorgegeben. Zumindest ein Refokussierungspuls wird ermittelt, der die vorgegebene Refokussierungspulsform aufweist. Die Magnetresonanzsequenz, die den Anregungspuls und den, insbesondere zeitlich darauffolgenden, zumindest einen Refokussierungspuls umfasst, wird bereitgestellt, beispielsweise in Form eines Sequenzdatensatzes.

## Beschreibung

Die Erfindung betrifft ein Verfahren zum Bereitstellen einer Magnetresonanzsequenz, eine Magnetresonanzvorrichtung und ein Computerprogrammprodukt.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Dabei wird ein Untersuchungsobjekt, beispielsweise ein Patient, in einem Untersuchungsbereich einer Magnetresonanzvorrichtung positioniert, in dem ein Hauptmagnet der Magnetresonanzvorrichtung ein Hauptmagnetfeld erzeugt. Während einer Magnetresonanzmessung werden gemäß einer Magnetresonanzsequenz hochfrequente (HF, engl. radio-frequency, RF) Sendepulse zur Erzeugung eines magnetischen Wechselfeldes (auch B1-Feld genannt) und Gradientenpulse zur Erzeugung eines magnetischen Feldgradienten, der dem Hauptmagnetfeld überlagert wird, in den Untersuchungsbereich eingestrahlt und damit Kernspins im Untersuchungsobjekt angeregt. Dadurch werden im Untersuchungsobjekt ortskodierte Echosignale ausgelöst, die oftmals auch Magnetresonanzsignale genannt werden. Die Magnetresonanzsignale können zur Rekonstruktion von Magnetresonanzabbildungen verwendet werden.

Gerade bei höheren Stärken des Hauptmagnetfelds, beispielsweise bei 7 Tesla, können die resultierenden Magnetfelder eine vergleichsweise hohe Inhomogenität aufweisen, welche sich als Artefakte in den Magnetresonanzabbildungen niederschlagen kann. Um dieses Problem zu reduzieren, können insbesondere die HF-Sendepulse angepasst werden, um ein B1-Feld hoher Homogenität zur erzeugen. Allerdings kann die Berechnung solch angepasster HF-Sendepulse sehr zeitaufwändig sein. Ferner wird ein Teil der eingestrahlten HF-Leistung durch das Untersuchungsobjekt absorbiert; dies wird durch die spezifische Absorptionsrate (SAR) beschrieben. Um den Patienten nicht zu gefährden, darf die SAR bestimmte Grenzwerte nicht überschreiten.

Aus Aufgabe der vorliegenden Erfindung kann angesehen werden, ein Verfahren zum Bereitstellen einer Magnetresonanzsequenz anzugeben, mit dem effizient Magnetresonanzsignale zur Generierung hochqualitativer Magnetresonanzabbildungen akquiriert werden können.

Die Aufgabe wird durch die Merkmale der unabhängigen Ansprüche gelöst. Vorteilhafte Ausgestaltungen sind in den abhängigen Ansprüchen beschrieben.

Demnach wird ein computerimplementiertes Verfahren zum Bereitstellen einer Magnetresonanzsequenz, insbesondere einer Spinecho-Sequenz, vorgeschlagen. Dabei wird ein Magnetfeldverteilungsdatensatz bereitgestellt. Zudem wird ein Anregungspuls ermittelt, der eine Anregungspulsform aufweist, wobei die Anregungspulsform in Abhängigkeit von dem Magnetfeldverteilungsdatensatz ermittelt wird. Ferner wird eine Refokussierungspulsform vorgegeben. Zumindest ein Refokussierungspuls wird ermittelt, der die vorgegebene Refokussierungspulsform aufweist. Die Magnetresonanzsequenz, die den Anregungspuls und den, insbesondere zeitlich darauffolgenden, zumindest einen Refokussierungspuls umfasst, wird bereitgestellt, beispielsweise in Form eines Sequenzdatensatzes.

Anregungspulse und Refokussierungspulse sind üblicherweise HF-Sendepulse, die vorzugsweise mittels einer Hochfrequenzantenneneinheit einer Magnetresonanzvorrichtung ausgesendet werden können. Die Hochfrequenzantenneneinheit kann eine oder mehrere Sendespulen, insbesondere Sendespulenelemente, umfassen, denen jeweils ein Sendekanal zugeordnet sein kann.

Die Magnetresonanzsequenz ist vorzugsweise eine Ansteuersequenz, die insbesondere für einen Zielbereich, beispielsweise eine Schicht, selektiv ist, zur Herstellung eines Zielanregungszustands, beispielsweise ein bestimmter, homogener Flipwinkel der Spins des Untersuchungsobjekts, für einen Erfassungsvorgang von Magnetresonanzsignalen eines Untersuchungsobjekts mit einer Magnetresonanzvorrichtung. Dabei umfasst die Magnetresonanzsequenz über zumindest einen Sendekanal, insbesondere mehrere Sendekanäle, einer Hochfrequenzantennenanordnung auszugebende Hochfrequenzpulse, insbesondere Anregungspulse und Refokussierungspulse.

Spinecho-Sequenzen sind weit verbreitete Sequenztypen auf dem Gebiet der MRT. Eine Spinecho-Sequenz umfasst üblicherweise eine Folge aus einem Anregungspuls, beispielsweise ein 90°-Puls, und zumindest einem Refokussierungspuls, beispielsweise ein 180°-Puls. Eine Spinecho-Sequenz kann insbesondere zur Erzeugung T1-gewichteter, Protonendichte-gewichteter oder stark T2-gewichteter Magnetresonanzabbildungen verwendet werden.

Bei einer Spinecho-Sequenz wird typischerweise ausgenutzt, dass ein Magnetresonanzsignal nach einem Zerfall eines FID-Signals wiedererscheint. Hierzu wird eine Dephasierung eines Kernspins (Quermagnetisierungszerfall) durch Einstrahlen eines Refokussierungspulses aufgehoben. Die Spins geraten wieder in Phase, und es entsteht das Spinecho zum Zeitpunkt TE (Echozeit).

Der Magnetfeldverteilungsdatensatz kann insbesondere eine B1-Feldkarte (engl. B1 field map), oder kurz: B1-Karte (engl. B1 map), umfassen. Vorzugsweise umfasst der Magnetfeldverteilungsdatensatz auch eine B0-Feldkarte (engl. B0 field map), oder kurz: B0-Karte (engl. B1 map).

Die B1-Karte beschreibt üblicherweise eine örtliche Verteilung eines B1-Feldes. Eine B1-Karte ist vorzugsweise eine, insbesondere räumliche, Repräsentation eines B1-Feldes. Das B1-Feld ist üblicherweise das durch eine Sendespule einer Magnetresonanzvorrichtung erzeugte magnetische Wechselfeld der HF-Einstrahlung. Die B0-Karte beschreibt üblicherweise eine örtliche Verteilung eines B0-Feldes. Eine B1-Karte ist vorzugsweise eine, insbesondere räumliche, Repräsentation eines B0-Feldes. Das B0-Feld ist üblicherweise das statische Hauptmagnetfeld der Magnetresonanzvorrichtung. Das B0-Feld wird üblicherweise durch einen, oftmals supraleitenden, Hauptmagneten der Magnetresonanzvorrichtung erzeugt. Die Repräsentation eines B1-Feldes bzw. eines B0-Feldes kann beispielsweise mittels eines Bildes erfolgen, in dem die Intensität eines Bild-Pixels oder Bild-Voxels die Stärke des B1-Feldes bzw. des B0-Feldes an dem Ort des Bild-Pixels oder Bild-Voxels darstellt.

Vorteilhafterweise beschreiben das B1- und/oder das B0-Feld die jeweilige Feldverteilung, wenn sich das Untersuchungsobjekt in einem Untersuchungsbereich der Magnetresonanzvorrichtung, insbesondere in einer Messposition, befindet. Insbesondere bei höheren Magnetfeldstärken können durch die Anwesenheit des Untersuchungsobjekts größere Inhomogenitäten der Felder auftreten. Vorteilhafterweise können solche Inhomogenitäten durch speziell angepasste HF-Pulse, insbesondere Anregungspulse und/oder Refokussierungspulse, reduziert werden.

Zur Erzeugung des Magnetfeldverteilungsdatensatzes kann eine Magnetresonanzmessung durchgeführt, insbesondere appliziert, werden, mit der Magnetresonanzsignale aufgenommen werden, anhand derer der Magnetfeldverteilungsdatensatz, insbesondere eine B1- und/oder B0-Karte, erzeugt werden kann. Es ist eine Reihe von Verfahren zur Erzeugung von B1- und/oder B0-Karten bekannt.

Die Anregungspulsform ist die Pulsform des Anregungspulses. Die Refokussierungspulsform ist die Pulsform des zumindest einen Refokussierungspulses. Die folgenden Ausführungen zu dem Begriff "Pulsform" beziehen sich insbesondere auf die Anregungspulsform und/oder Refokussierungspulsform. Die folgenden Ausführungen zu dem Begriff "Puls" beziehen sich insbesondere auf den Anregungspuls und/oder Refokussierungspuls.

Eine Pulsform (also insbesondere Anregungspulsform bzw. Refokussierungspulsform) ist insbesondere das Profil und/oder die Gestalt eines Pulses (also insbesondere Anregungspuls bzw. Refokussierungspuls). Eine Pulsform kann insbesondere durch eine Hüllkurve des Pulses, insbesondere im Basisband, beschrieben werden. Die Pulsform ist insbesondere unabhängig von einer absoluten Höhe, insbesondere Amplitude, und/oder einer absoluten Breite, insbesondere Länge, des Pulses.

Eine Pulsform eines Pulses kann insbesondere durch einen zeitlichen Verlauf des Pulses beschrieben werden, z.B. ausgedrückt als ein Pulsprofil S(t), wobei das Pulsprofil S(t) insbesondere durch eine komplexwertige Funktion beschrieben werden kann und t die Zeit repräsentiert.

Insbesondere ist möglich, dass eine Pulsform eines Pulses durch einen zeitlichen Verlauf eines, insbesondere reellwertigen, Momentanwertes und/oder einer Auslenkung des Pulses und/oder einer Phase des Pulses beschrieben werden kann. Beispielsweise können Funktionen von Pulsen, insbesondere Sinc-Pulsen, negative Werte aufweisen, die dann aber als positive Amplitudenwerte mit 180°-Phasenversatz im Phasenverlauf dargestellt werden können.

Vorzugsweise ist die Pulsform, insbesondere deren Amplitude und/oder Breite, normiert. Vorzugsweise ist die Pulsform eine normierte HF-Pulsform. Dies kann mathematisch beispielsweise mit S(t) = nS(t) ausgedrückt werden.

Ein Puls kann insbesondere mittels eines Skalierungsfaktors beschrieben werden. Ein Puls P selbst kann beispielsweise durch eine Multiplikation eines Skalierungsfaktors F mit dem Pulsprofil S(t) beschrieben werden: P = F · S(t). Der Skalierungsfaktor F kann ein Skalar sein. Der Skalar kann komplexwertig sein, insbesondere abhängig von einer Phase sein. Der Skalierungsfaktor F kann insbesondere als Shimkoeffizient, insbesondere zum Shimmen eines B1-Feldes, fungieren.

Ein Puls kann insbesondere mittels eines Längenskalierungsfaktors beschrieben werden. Ein Puls P selbst kann beispielsweise durch eine Division der Zeitvariable t mit einem Längenskalierungsfaktor T beschrieben werden: P ~ S(t/T). Der Längenskalierungsfaktor T kann insbesondere eine für den Puls charakteristische Zeitdauer sein.

Die Pulsform kann insbesondere mit einem Zeit-Bandbreitenprodukt (engl. time-bandwidth, TBW, product) beschrieben werden. Je nach gewünschter Bandbreite, insbesondere Frequenz-Bandbreite, ergibt sich damit bei festem TBW-Produkt die zeitliche Länge T des Pulses. Beispielsweise kann bei, insbesondere für schichtselektive Anregung verwendeten, Sinc-Pulsen die Pulsform mit einem Zeit-Bandbreitenprodukt beschrieben werden. Insbesondere entspricht die zeitliche Länge T des Pulses einer Anzahl an Nulldurchgängen eines Sinc-Pulses.

Ein Puls kann insbesondere mittels eines Skalierungsfaktors und eines Breitenskalierungswertes beschrieben werden. Ein Puls P selbst kann beispielsweise durch eine Multiplikation eines Skalierungsfaktors F mit dem Pulsprofil S(t) und durch eine Division der Zeitvariable t mit einem Breitenskalierungswertes T beschrieben werden: P = F · S(t/T).

Die Pulsform und/oder Amplitude und/oder Phase eines HF-Sendepulses kann beispielsweise einer Form und/oder Amplitude und/oder Phase eines Spannungspulses, der an die jeweilige Sendespule angelegt wird, und/oder eines Strompulses, der durch die Sendespule fließt, entsprechen.

Vorzugsweise ist der Anregungspuls ein paralleler Anregungspuls und der zumindest eine Refokussierungspuls zumindest ein paralleler Refokussierungspuls. Unter parallelen Pulsen sind insbesondere Pulse zu verstehen, die gleichzeitig über mehrere Sendekanäle appliziert werden. Insbesondere kann unter einem parallelen Puls auch ein pTx-Puls verstanden werden.

Vorteilhafterweise erfolgt die Ermittlung des Anregungspulses, insbesondere der Anregungspulsform, in Abhängigkeit von dem Magnetfeldverteilungsdatensatz, insbesondere in Abhängigkeit von einer B1-Karte, derart, dass eine durch den Anregungspulses erzeugte Flipwinkelverteilung (während einer möglichen Magnetresonanzmessung gemäß der bereitgestellten Magnetresonanzsequenz) möglichst homogen ist. Vorzugsweise wird dazu für jeden Sendekanal die Anregungspulsform, insbesondere das Anregungspulsprofil, des Anregungspulses auf Basis von B1-Karten und B0-Karten berechnet. Vorzugsweise wird ferner für jede Gradientenspule der Gradientenspuleneinheit eine Gradientenpulsform berechnet. Vorteilhafterweise werden (während einer Magnetresonanzmessung) parallel Anregungspulse mit den berechneten Anregungspulsformen und Gradientenpulse mit den berechneten Gradientenpulsformen appliziert, insbesondere um eine möglichst homogene Flipwinkelverteilung zu erzeugen.

Als Flipwinkel wird üblicherweise ein Anregungswinkel für eine Pulssequenz bezeichnet. Es ist üblicherweise der Winkel, bezogen auf die Richtung des Hauptmagnetfeldes, in den eine Nettomagnetisierung durch Applizieren eines HF-Pulses bei der Larmor-Frequenz gedreht wird. In Spinecho-Sequenzen werden beispielsweise ein 90°-Puls und eine Reihe von 180°-Pulsen verwendet.

Eine solche Technik ist auch als B1-Shimmung oder HF-Shimming bekannt. Vorteilhafterweise können aus Magnetresonanzsignalen, die bei Vorliegen einer homogenen B1-Feldverteilung akquiriert wurden, hochqualitative Magnetresonanzabbildungen rekonstruiert werden.

Vorteilhafterweise erfolgt die Ermittlung des Anregungspulses, insbesondere der Anregungspulsform, in Abhängigkeit von dem Magnetfeldverteilungsdatensatz, insbesondere in Abhängigkeit von einer B1-Karte, derart, dass eine durch den Anregungspulses bewirkte SAR-Exposition möglichst gering ist. Vorteilhafterweise kann dadurch die Belastung eines Patienten durch die während einer Magnetresonanzmessung absorbierte HF-Leistung reduziert werden.

Vorteilhafterweise kann der Anregungspuls freier parametrisiert werden als der zumindest eine Refokussierungspuls, da die Pulsform des Anregungspuls (im Gegensatz zur Pulsform des Refokussierungspulses) nicht vorgegeben ist. Die erzielbare bessere Homogenität der komplexen Flipwinkelverteilung, die am Anfang durch den Anregungspuls erzeugt wird, wirkt sich dann positiv auch auf auf den zumindest einen Refokussierungspuls folgende Spinechos (und gegebenenfalls stimulierte Echos) aus. Insbesondere kann diese erzielbare bessere räumliche Homogenität des Magnetresonanzsignals über mehrere Spinechos hinweg erhalten bleiben.

Die vorgegebene Refokussierungspulsform ist insbesondere unabhängig von dem Magnetfeldverteilungsdatensatz. Der bereitgestellte Magnetfeldverteilungsdatensatz hat also keinen Einfluss auf die Refokussierungspulsform. Die vorgegebene Refokussierungspulsform ist insbesondere unabhängig von einem mittels der bereitgestellten Magnetresonanzsequenz zu untersuchenden Untersuchungsobjekt. Die Refokussierungspulsform wird beispielsweise im Vorfeld einer (diagnostischen) Magnetresonanzmessung, beispielsweise bei einer Sequenzentwicklung, festgelegt. Vorteilhafterweise braucht die Refokussierungspulsform für die Bereitstellung der Magnetresonanzsequenz, insbesondere für eine spezifische Magnetresonanzmessung gemäß der bereitgestellten Magnetresonanz, nicht neu ermittelt zu werden. Dies kann eine bedeutende Zeitersparnis bewirken, so dass die Bereitstellung der Magnetresonanzsequenz effizient durchgeführt werden kann.

Beispielsweise kann mit einer Magnetresonanzvorrichtung zunächst eine Vormessung zur Ermittlung des Magnetfeldverteilungsdatensatzes durchgeführt werden. Insbesondere beschreiben die in der Vormessung aufgenommenen Daten zumindest einen Teil des Untersuchungsobjekts und/oder bilden es ab. Dann kann die Magnetresonanzsequenz bereitgestellt werden, wobei das Vorgeben der Refokussierungspulsform, das Ermitteln des Anregungspulses, das Ermitteln des zumindest einen Refokussierungspulses und das Bereitstellen der Magnetresonanzsequenz beispielsweise mittels einer Systemsteuereinheit der Magnetresonanzvorrichtung durchgeführt werden kann. Die Systemsteuereinheit kann dafür beispielsweise eine Berechnungseinheit und/oder eine Speichereinheit aufweisen. Gemäß der bereitgestellten Magnetresonanzsequenz kann eine Hauptmessung durchgeführt werden. Die Hauptmessung ist vorzugsweise geeignet, diagnostisch auswertbare und/oder bildgebende Magnetresonanzsignale zu erfassen. Anhand der in der Hauptmessung akquirierten Magnetresonanzsignale können insbesondere eine oder mehrere Magnetresonanzabbildungen erzeugt werden.

Eine mögliche Ausführungsform des Verfahrens sieht vor, dass der Anregungspuls ein dynamischer Puls ist. Als dynamischer Puls kann insbesondere ein HF-Sendepuls angesehen werden, dessen Phase und/oder Amplitude sich im zeitlichen Verlauf des Pulses ändert, während mittels einer Gradientenspuleneinheit der Magnetresonanzvorrichtung eine, insbesondere vorbestimmte, Gradiententrajektorie abgetastet wird. Insbesondere erfolgt die Abtastung der Gradiententrajektorie zeitlich mit der Variation von Phase und/oder Amplitude des HF-Sendepulses.

Die Gesamtheit aus HF-Sendepuls und Gradiententrajektorie kann auch als dynamischer Puls betrachtet werden. Dann wäre der HF-Sendepuls ein Teil des dynamischen Pulses.

Die zumindest eine Pulsform und/oder Amplitude und/oder Phase des HF-Sendepulses bzw. eines Teilpulses kann beispielsweise einer Form und/oder Amplitude und/oder Phase eines Spannungspulses, der an die jeweilige Sendespule angelegt wird, und/oder eines Strompulses, der durch die Sendespule fließt, entsprechen.

Die zumindest eine Pulsform und/oder Amplitude und/oder Phase des Gradientenpulses kann beispielsweise einer Form und/oder Amplitude und/oder Phase eines Spannungspulses, der an die Gradientenspuleneinheit angelegt wird, und/oder eines Strompulses, der durch die Gradientenspuleneinheit fließt, entsprechen.

Durch einen dynamischen Puls kann vorteilhafterweise das dadurch erzeugte B1-Feld präziser kontrolliert werden. Insbesondere können mit einem pTx-Puls Magnetfeldinhomogenitäten ausgeglichen werden, das vor allem bei höheren Feldstärken des Hauptmagnetfelds ab 5, insbesondere 7, Tesla besonders vorteilhaft sein kann. Gerade bei hohen Feldstärken kann ein dynamisches paralleles Senden von Vorteil sein, weil die dort auftretenden B1-Effekte oftmals so kurzwellig sind, dass sie innerhalb einer Magnetresonanzabbildung sichtbar wären.

Eine mögliche Ausführungsform des Verfahrens sieht vor, dass der Anregungspuls ein pTx-Puls ist. "pTx" steht hierbei für "paralleles Senden", engl. "parallel transmission". Ein pTx-Puls kann mehrere Teilpulse umfassen, die parallel, insbesondere gleichzeitig, durch jeweils eine Sendespule einer Hochfrequenzantenneneinheit der Magnetresonanzvorrichtung gesendet werden. Jeder Sendespule kann wiederum ein Sendekanal zugeordnet werden. Dabei können sich die Teilpulse insbesondere in ihrer Pulsform und/oder Amplitude und/oder Phase unterscheiden. Ferner können die Teilpulse eine zeitliche Verzögerung zueinander aufweisen. Beispielsweise setzt sich ein emittierbarer HF-Sendepuls aus mehreren Teilpulsen zusammen, die voneinander abweichen und jeweils durch eine Sendespule einer mehrkanaligen Sendespulenanordnung der Hochfrequenzantenneneinheit gesendet werden können. Vorzugsweise sind zumindest ein Teil der mehreren Teilpulse, insbesondere alle Teilpulse, dynamische Pulse.

Bei einem Senden eines pTx-Pulses lässt sich vorteilhafterweise eine vorbestimmte räumliche Verteilung der Anregung als zusätzlicher Freiheitsgrad durch Interferenz der Signale der Mehrzahl an Sendekanälen über eine Mehrzahl an Sendespulen der Hochfrequenzantenneneinheit erzielen, die beim Bestimmen des pTx-Pulses beispielsweise durch Variation von Phase und Amplitude eingestellt wird.

Eine mögliche Ausführungsform des Verfahrens sieht vor, dass die Magnetresonanzsequenz für mehrere Sendekanäle jeweils einen Anregungspuls und zumindest einen Refokussierungspuls umfasst. Vorzugsweise ist vorgesehen, dass die jeweiligen Anregungspulse bzw. Refokussierungspuls parallel, insbesondere gleichzeitig, appliziert werden.

Die Magnetresonanzsequenz kann beispielsweise vorgesehen sein, mit einer Magnetresonanzvorrichtung appliziert zu werden, die N Sendekanäle umfasst. Vorzugsweise werden gemäß der Magnetresonanzsequenz parallel N Anregungspulse (also ein Anregungspuls pro Sendekanal), appliziert, dann parallel N erste Refokussierungspulse (also ein Refokussierungspuls pro Sendekanal) und möglicherweise noch weitere N Refokussierungspulse. Diese Applikation der N Anregungspulse, N erster (und gegebenenfalls weiterer) Refokussierungspulse erfolgt insbesondere pro aufzunehmende Schicht.

Vorzugsweise ist der zumindest eine Refokussierungspuls ein statischer pTx-Puls ("statisch" weil feste Pulsform aufweisend, so dass damit nur ein B1-Feld erzeugt wird) und der Anregungspuls ein dynamischer pTx-Puls ("dynamisch" weil kanalspezifische Pulsformen aufweisend, so dass während eines Pulses ständig verschiedene B1-Felder erzeugt werden können).

Statisches pTx besteht vorzugsweise darin, allen Sendespulen mit der gleichen HF-Pulsform (z.B. Rechteck- oder Sinc-Form) anzusteuern, diese aber mit spulenspezifischen Magnituden und Phasen zu skalieren. Dadurch kann ein (statisches) Anregungsfeld erzeugt werden, welches räumlich etwas homogener sein kann als ein unkalibriertes Anregungsfeld. Dynamisches pTx erzeugt vorzugsweise zeitlich veränderliche, jeweils für sich inhomogene B1-Felder, die gleichzeitig mit ebenso zeitlich veränderlichen B0-Gradientenfeldern, oft beschrieben als "Sende-k-Raum Trajektorie", angelegt werden. So können sehr homogene Flipwinkelverteilungen am Ende des dynamischen pTx-Pulses erreicht werden.

Eine mögliche Ausführungsform des Verfahrens sieht vor, dass die Ermittlung der Anregungspulsform eine Optimierung einer Homogenität einer durch den Anregungspuls erzeugbaren, insbesondere zu erzeugenden, Flipwinkelverteilung umfasst. Vorzugsweise erfolgt die Optimierung anhand des Magnetfeldverteilungsdatensatzes. Vorzugsweise wird der Anregungspuls, insbesondere seine Pulsform, anhand des zumindest einen Magnetfeldverteilungsdatensatzes hinsichtlich einer maximalen Homogenität der Flipwinkelverteilung optimiert. Vorteilhafterweise steht zur Optimierung der Flipwinkelverteilung jede beliebige Pulsform zur Verfügung. (Hingegen weist der zumindest eine Refokussierungspuls eine vorgegebene Pulsform auf.)

Es ist denkbar, dass die Ermittlung der Anregungspulsform eine Optimierung einer Homogenität der Flipwinkelverteilung in einer (aufzunehmenden) Schicht umfasst. Eine mögliche Ausführungsform sieht vor, dass die Ermittlung der Anregungspulsform eine Optimierung einer Homogenität der Flipwinkelverteilung in nur einem, insbesondere zentralen, Teilbereich einer Schicht umfasst. Vorteilhafterweise liegt in diesem Teilbereich ein aufzunehmendes Körperteil des Untersuchungsobjekts.

Insbesondere kann man den, insbesondere dynamischen, Anregungspuls so optimieren, dass er nicht die ganze Schicht, beispielsweise in xy-Richtung, anregt, sondern beispielsweise nur einen kleinen rechteckigen Ausschnitt in der Mitte, in dem beispielsweise eine Prostata des Patienten liegt. Dann wird auch durch die Refokussierungspulse außerhalb idealerweise nichts oder nur wenig angeregt und muss dann auch nicht ortskodiert werden. Vorteilhafterweise schneller so das aufzunehmende Körperteil (ohne alles drum herum) schneller aufgenommen werden.
Vorteilhafterweise steht beim Anregungspuls für die Optimierung der Homogenität, neben etwaigen weiteren Parametern, auch die Pulsform des Anregungspuls als anpassbarer Parameter zur Verfügung. (Im Gegensatz dazu ist die Pulsform des zumindest einen Refokussierungspulses fest vorgegeben, d.h. stünde für etwaige Optimierungszwecke nicht zur Verfügung.)

Eine mögliche Ausführungsform des Verfahrens sieht vor, dass Anregungspuls und/oder der zumindest eine Refokussierungspulses räumlich selektiv, insbesondere schichtselektiv, sind. Die räumliche Selektion kann insbesondere eine Selektion einer Schicht betreffen, aber sind auch andere räumliche Bereiche und/oder Geometrien denkbar, beispielsweise eine Kugel. Vorzugsweise weist die Anregungspulsform und/oder die die Refokussierungspulsform eine Fourier-Transformierte auf, die einem gewünschten räumlichen Selektion, insbesondere einem gewünschten örtlichen Anregungsprofil, entspricht.

Vorzugsweise sieht die zu bereitstellende Magnetresonanzsequenz vor, dass zeitgleich mit dem Anregungspuls und/oder der zumindest einen Refokussierungspuls ein Selektionsgradient, insbesondere Schichtselektionsgradienten, appliziert wird. Vorteilhafterweise wird dadurch im Untersuchungsbereich ein magnetischen Feldgradient erzeugt, so dass gemäß dem Frequenzinhalt des Anregungspulses und/oder des zumindest einen Refokussierungspuls ein damit korrespondierender räumlicher Bereich angeregt wird.

Vorteilhafterweise wird als Refokussierungspulsform eine Pulsform vorgegeben, die eine gewünschte räumliche Selektion, beispielsweise eine Schichtselektion, bewirkt. Vorteilhafterweise weist die Refokussierungspulsform eine räumlich besonders klar definierte Wirkung auf. Beispielsweise wird ein räumlicher Bereich vorgegeben, beispielsweise eine Schicht oder ein anderes Volumen, für den der Refokussierungspuls selektiv sein soll.

Bei Vorliegen eines magnetischen Feldgradienten weist ein bestimmter räumlicher Bereich, insbesondere eine bestimmte Schicht, eine Magnetfeldstärke B auf, die in einem bestimmten Bereich des Magnetfeldes B liegt, z.B. zwischen Bₐ und B_{b}. Über die physikalische Beziehung f = γ · B, wobei γ eine kernspezifische gyromagnetische Konstante ist, ist die Frequenz f mit dem Magnetfeld verknüpft. Somit ist bekannt, welchen Frequenzinhalt, insbesondere Frequenzbandbreite, ein entsprechender HF-Puls, insbesondere der Refokussierungspuls aufweisen sollte. Durch eine Fouriertransformation vom Frequenzraum bzw. Ortsraum in den Zeitraum kann eine Pulsform bestimmt werden, die möglichst exakt einem bestimmten räumlichen Bereich entspricht.

Vorteilhafterweise kann als Refokussierungspulsform von vorneherein eine Pulsform vorgegeben werden, die einem bestimmten Anregungsprofil, beispielsweise einer Schichtanregung, entspricht, ohne dass eine zeitaufwändige Neuberechnung für eine gerade durchzuführende Magnetresonanzmessung notwendig ist.

Wenn der Anregungspuls vor allem dahingehend optimiert ist, eine möglichst hohe Homogenität des B1-Feldes zu bewirken, kann diese Nebenbedingung zur Folge haben, dass die räumliche Selektion, beispielsweise Schichtselektion, mangelhaft ist. Zudem können Ungenauigkeiten in der Kalibrierung (beispielsweise durch ungenaue B1-Karten und/oder B0-Karten und/oder durch eine ungenaue Kalibrierung der Verstärkung der Sendekanäle) werden solche Pulse anfällig sind für eine schlechtere räumliche Selektion.

Vorteilhafterweise "schneiden" Refokussierungspulse mit selektionsoptimierter Refokussierungspulsform das gewünschte räumliche Profil, insbesondere Schichtprofil, aus einem möglicherweise mangelhaften räumlichen Profil, insbesondere Schichtprofil, des Anregungspulses heraus und stellen so die gewünschte Selektion, insbesondere Schichtselektion, sicher. Durch die Kombination eines Anregungspuls mit freier Pulsform und einem oder mehreren Refokussierungspulsen mit vorgegebener Pulsform, die hinsichtlich des Anregungsprofils optimiert ist, kann insbesondere eine hohe B1-Homogenität bei gleichzeitig präziser räumlicher Anregung erreicht werden.

Eine mögliche Ausführungsform des Verfahrens sieht vor, dass die Refokussierungspulsform eine Form eines Sinc-Pulses und/oder eines SLR-Pulses und/oder eines Rechteck-Pulses aufweist.

Die Form eines Sinc-Pulses kann insbesondere mathematisch beschrieben werden durch eine mittels einer Sinc-Funktion (sinc(x) = sin (x)/x). Theoretisch würde eine solche Funktion eine perfekte Schichtselektion ermöglichen. Allerdings würde ein (unangepasster) Sinc-Puls eine unendliche Anzahl von Nebenkeulen (und damit eine unendliche Übertragungszeit) erfordern. Vorteilhafterweise wird der Sinc-Impuls daher modifiziert, beispielsweise mittels eines Filters. Insbesondere kann der Sinc-Pulse mittels einer Fensterfunktion adaptiert sein, um die Pulslänge auf ein gewünschtes Maß zu reduzieren. Eine mögliche Fensterfunktion ist beispielsweise die Hamming-Fensterfunktion.

SLR steht für "Shinnar-Le Roux"; SLR-Pulse können mittels eines SLR-Algorithmus bestimmt werden. Vorteilhafterweise weisen SLR-Pulse sehr gute Schichtprofile auf mit keinen Seitenkeulen und sehr kleinen Welligkeiten außerhalb der Schicht.

Eine mögliche Ausführungsform des Verfahrens sieht vor, dass die Ermittlung des zumindest eines Refokussierungspulses in Abhängigkeit von dem Magnetfeldverteilungsdatensatz erfolgt. Die Ermittlung umfasst vorteilhafterweise eine Optimierung einer Homogenität einer durch den Refokussierungspuls erzeugbaren, insbesondere zu erzeugenden, Magnetfeldverteilung (insbesondere B1-Feldverteilung).

Auch wenn die Pulsform des zumindest einen Refokussierungspulses vorgegeben ist, können vorteilhafterweise andere Parameter des Refokussierungspulses an den Magnetfeldverteilungsdatensatz angepasst werden. Vorteilhafterweise kann dadurch eine bessere Homogenität des durch den zumindest einen Refokussierungspulse erzeugbaren, insbesondere zu erzeugenden, Magnetfeldverteilung (insbesondere B1-Feldverteilung) erreicht werden als ohne Anpassung.

Eine mögliche Ausführungsform des Verfahrens sieht vor, dass das Ermitteln des zumindest eines Refokussierungspulses eine Ermittlung eines Skalierungsfaktors in Abhängigkeit von dem Magnetfeldverteilungsdatensatz umfasst, wobei der Refokussierungspuls anhand der (vorgegebenen) Refokussierungspulsform und dem Skalierungsfaktor ermittelt wird. Vorzugsweise wird für jeden Refokussierungspuls ein eigener Vektor mit entsprechenden Koeffizienten, insbesondere einer pro Sendekanal, berechnet. Eine solche Berechnung kann beispielsweise mit dem in Malik SJ, Beqiri A, Padormo F, Hajnal JV. Direct signal control of the steady-state response of 3D-FSE sequences. Magn Reson Med. 2015 Mar;73(3):951-63. doi: 10.1002/mrm.25192. Epub 2014 Mar 17. PMID: 24639096; PMCID: PMC7614097. beschriebenen DSC-Ansatz ("DSC" für Direct Signal Control) erfolgen.

Der Skalierungsfaktor ist vorzugsweise ein, insbesondere komplexwertiger, Skalar. Insbesondere kann der Skalierungsfaktor ein Koeffizient einer mathematischen Repräsentation der Refokussierungspulsform sein. Insbesondere kann der Skalierungsfaktor einen Shim-Koeffizienten repräsentieren. Die Ermittlung des Refokussierungspuls anhand der Refokussierungspulsform und dem Skalierungsfaktor kann beispielsweise eine Multiplikation des Skalierungsfaktors F_{RP} mit einer mathematischen Repräsentation der Refokussierungspulsform, beispielsweise S_{RP}(t) umfassen, beispielsweise PRₚ = FRₚ ·S_{RP}(t).

Eine mögliche Ausführungsform des Verfahrens sieht vor, dass die Magnetresonanzsequenz ein Senden des Anregungspuls und des zumindest einen Refokussierungspulses mittels mehrerer Sendekanäle vorsieht, wobei für jeden Sendekanal ein kanalspezifischer Skalierungsfaktor ermittelt wird.

Wenn die Magnetresonanzsequenz beispielsweise ein Senden des Anregungspuls und des zumindest einen Refokussierungspulses mittels N Sendekanälen vorsieht, werden N Skalierungsfaktor ermittelt, also für jeden der N Sendekanäle jeweils ein Skalierungsfaktor.

Die kanalspezifischen Skalierungsfaktoren können beispielsweise in Form eines Vektors, insbesondere eines Spaltenvektors, repräsentiert werden. Beispielsweise solche ein Vektor N Koordinaten umfassen, wobei jede der N Koordinaten einen kanalspezifischen Skalierungsfaktor umfasst, insbesondere aus einem kanalspezifischen Skalierungsfaktor besteht.

Eine mögliche Ausführungsform des Verfahrens sieht vor, dass der zumindest eine Refokussierungspuls mehrere Refokussierungspulse umfasst. Vorteilhafterweise werden mittels der mehreren Refokussierungspulse auch mehrere Spinechos erzeugt. Vorteilhafterweise kann die durch den Anregungspuls erzielbare bessere Homogenität über die mehreren Spinechos hinweg erhalten bleiben.

Vorteilhafterweise wird innerhalb einer anzuregenden Schicht durch den Anregungspuls eine homogener Flipwinkel und eine entsprechende Phase der Kernspins so erzeugt, dass darauffolgende, insbesondere sinc-förmige, insbesondere mit einem geeigneten Skalierungsfaktor skalierte, Refokussierungspulse im Wesentlichen dasselbe Phasenprofil erzeugen.

Vorteilhafterweise bewirken die Skalierungsfaktoren einen B1-Shim bzw. ein geshimmtes B1-Feld. Wenn beispielsweise allen kanalspezifischen Skalierungsfaktoren bzw. Shim-Koeffizienten eine 90°-Phase hinzuaddiert wird, weist auch das resultierende B1-Feld eine Änderung um 90° in seiner räumlichen Phasenverteilung auf. Vorteilhafterweise unterscheidet sich dann die durch den zumindest einen Refokussierungspuls bewirkte Phasenverteilung gegenüber der durch den Anregungspuls bewirkte Phasenverteilung um 90°.

Insbesondere kann die die Magnetresonanzsequenz mehrere Echozüge umfassen, wobei für jeden Echozug, ein Anregungspuls und zumindest ein Refokussierungspuls gemäß einem vorangehend beschriebenen Verfahren ermittelt wird.

Vorzugsweise ist die die Magnetresonanzsequenz eine RARE-Sequenz (Rapide Acquisition with Relaxation Enhancement), insbesondere eine TSE-Sequenz (Turbo Spin Echo) und/oder FSE-Sequenz (Fast Spin Echo). Solche Sequenzen umfassen üblicherweise einen oder mehrere Echozüge mit jeweils einem Anregungspuls und mehreren Refokussierungspulsen.

Ferner wird ein computerimplementiertes Verfahren zur Bereitstellung einer Gesamtmagnetresonanzsequenz vorgeschlagen, die mehrere gemäß einem vorangehend beschriebenen Verfahren zur zum Bereitstellen einer Magnetresonanzsequenz bereitgestellte Magnetresonanzsequenzen umfasst.

Ferner wird eine Magnetresonanzvorrichtung vorgeschlagen, die ausgebildet ist, ein vorangehend beschriebenes computerimplementiertes Verfahren zum Bereitstellen einer Magnetresonanzsequenz bzw. Gesamtmagnetresonanzsequenz auszuführen. Vorzugsweise umfasst die Magnetresonanzvorrichtung eine Recheneinheit, beispielsweise umfassend einen oder mehrere Prozessoren, und/oder eine Speichereinheit, beispielsweise umfassend ein oder mehrere Speichermodule, um das Verfahren auszuführen.

Ferner wird ein Computerprogrammprodukt vorgeschlagen, das ein Programm umfasst und direkt in einen Speicher einer programmierbaren Systemsteuereinheit einer Magnetresonanzvorrichtung ladbar ist und Programmmittel, z.B. Bibliotheken und Hilfsfunktionen, aufweist, um ein vorgeschlagenes Verfahren zum Bereitstellen einer Magnetresonanzsequenz bzw. Gesamtmagnetresonanzsequenz auszuführen, wenn das Computerprogrammprodukt in der Systemsteuereinheit der Magnetresonanzvorrichtung ausgeführt wird. Das Computerprogrammprodukt kann dabei eine Software mit einen Quellcode, der noch kompiliert und gebunden oder der nur interpretiert werden muss, oder einen ausführbaren Softwarecode umfassen, der zur Ausführung nur noch in die Systemsteuereinheit zu laden ist.

Durch das Computerprogrammprodukt kann das vorgeschlagene Verfahren vorteilhafterweise schnell, identisch wiederholbar und robust ausgeführt werden. Das Computerprogrammprodukt ist vorzugsweise so konfiguriert, dass es mittels der Systemsteuereinheit die vorgeschlagenen Verfahrensschritte ausführen kann. Die Systemsteuereinheit weist dabei jeweils die Voraussetzungen wie beispielsweise einen entsprechenden Arbeitsspeicher, eine entsprechende Grafickarte oder eine entsprechende Logikeinheit auf, so dass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor einer lokalen Systemsteuereinheit geladen werden kann, der mit der Magnetresonanzvorrichtung direkt verbunden oder als Teil der Magnetresonanzvorrichtung ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Systemsteuereinheit einer Magnetresonanzvorrichtung ein vorgeschlagenes Verfahren durchführen.

Beispiele für elektronische lesbare Datenträger sind eine DVD, ein Magnetband oder einen USB-Stick, auf welchem elektronisch lesbare Steuerinformationen, insbesondere Software, gespeichert ist. Wenn diese Steuerinformationen von dem Datenträger gelesen und in eine Systemsteuereinheit der Magnetresonanzvorrichtung gespeichert werden, können alle vorgeschlagenen Ausführungsformen der vorab beschriebenen Verfahren durchgeführt werden.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine Magnetresonanzvorrichtung,
- Fig. 2: ein Ablauf eines Verfahrens zum Bereitstellen einer Magnetresonanzsequenz,
- Fig. 3: ein Echozug einer Magnetresonanzsequenz mit einem Anregungspuls mit freier Pulsform und mehreren Refokussierungspulsen mit vorgegebener Pulsform,
- Fig. 4: mehrere Echozüge einer Gesamtmagnetresonanzsequzenz,
- Fig. 5: mehrere Echozüge mehrerer Sendekanäle.

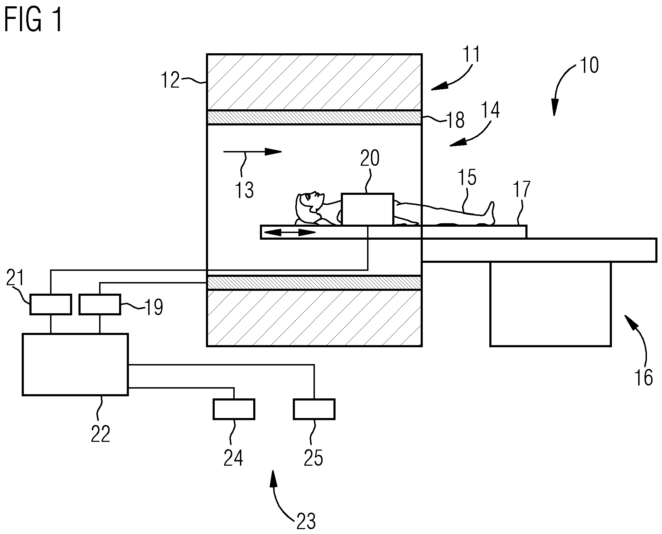

In Fig. 1 ist eine Magnetresonanzvorrichtung 10 schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13, insbesondere eines B0-Feldes, aufweist. Zudem umfasst die Magnetresonanzvorrichtung 10 einen Patientenaufnahmebereich 14 zu einer Aufnahme eines Untersuchungsobjekts wie eines Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als Bestandteil einer Lokalspule 20 ausgebildet ist. Lokalspulen sind üblicherweise direkt am Patienten 15 angeordnet. Grundsätzlich kann die Hochfrequenzantenneneinheit 20 aber auch insbesondere als fest in die Magnetresonanzvorrichtung 10 integrierte Körperspule ausgebildet sein. Gerade bei Magnetresonanzvorrichtungen, die eine hohe Hauptmagnetfeldstärke aufweisen, z.B. 7 Tesla, eigenen sich lokale Sendeanordnungen oft besser.

Die Hochfrequenzantenneneinheit 20 wird von einer Hochfrequenzantennensteuereinheit 21 der Magnetresonanzvorrichtung 10 gesteuert und strahlt HF-Pulse in einen Untersuchungsraum ein, der im Wesentlichen von einem Patientenaufnahmebereich 14 der Magnetresonanzvorrichtung 10 gebildet ist. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale erzeugt. Die Hochfrequenzantenneneinheit 20 ist zum Empfang der Magnetresonanzsignale ausgebildet.

Die Hochfrequenzantenneneinheit 20 kann mehrere Sendespulen, insbesondere Spulenelemente, umfassen, die jeweils ausbildet sind, einen HF-Puls zu erzeugen; ein solcher HF-Puls kann insbesondere ein Teilpuls eines dynamischen Pulses und/oder pTx-Pulses sein. Jede dieser mehreren Sendespulen kann einem Sendekanal zugeordnet sein. Solch ein Sendekanal kann insbesondere weitere sendekanalspezifische Komponenten umfassen, wie beispielsweise einen Signalverstärker. Solche Komponenten können insbesondere Bestandteil der Hochfrequenzantennensteuereinheit 21 sein.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer bildgebenden Magnetresonanzsequenz, insbesondere einer Spin-Echo-Sequenz. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

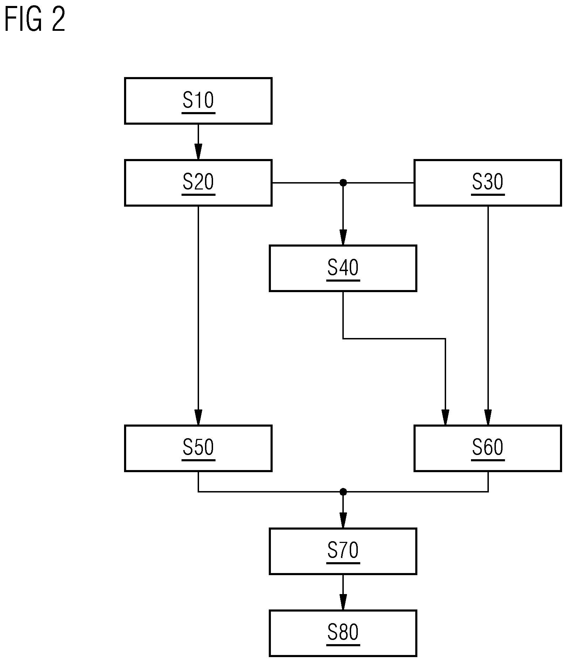

In Fig. 2 ist ein möglicher Ablauf eines computerimplementierten Verfahrens zum Bereitstellen einer Magnetresonanzsequenz, insbesondere einer Spin-Echo-Sequenz, dargestellt. Die damit bereitgestellte Magnetresonanzsequenz kann beispielsweise mit einer in Fig. 1 gezeigten Magnetresonanzvorrichtung 10 ausgeführt werden.

Gemäß der dargestellten Ausführungsform wird S10 wird mit der Magnetresonanzvorrichtung 10 ein Magnetfeldverteilungsdatensatz aufgenommen und in S20 bereitgestellt. Der Magnetfeldverteilungsdatensatz kann insbesondere eine B1-Feldkarte und/oder eine B0-Feldkarte umfassen.

In S30 wird eine Refokussierungspulsform vorgegeben. Vorzugsweise wird in S40 wird für zumindest einen der Refokussierungspulse, insbesondere für jeden Refokussierungspuls, ein Skalierungsfaktor in Abhängigkeit von dem in S20 bereitgestellten Magnetfeldverteilungsdatensatz ermittelt. Der Refokussierungspuls wird dann vorzugsweise in S50 anhand der in S30 vorgegebenen Refokussierungspulsform und dem in S40 ermittelten Skalierungsfaktor ermittelt.

Falls die Magnetresonanzsequenz ein Senden des Anregungspuls und des zumindest einen Refokussierungspulses mittels mehrerer Sendekanäle vorsieht, insbesondere falls die Hochfrequenzantenneneinheit 20 mehrere Sendespulen umfasst, kann für jeden Sendekanal ein kanalspezifischer Skalierungsfaktor ermittelt werden. Vorzugsweise umfasst ein Refokussierungspuls mehrere kanalspezifische Teilpulse, die vorgesehen sind, parallel, insbesondere gleichzeitig, gesendet zu werden.

In S50 wird ein Anregungspuls ermittelt, der eine Anregungspulsform aufweist, wobei die Anregungspulsform in Abhängigkeit von dem Magnetfeldverteilungsdatensatz ermittelt wird. Der Anregungspuls ist vorzugsweise ein dynamischer Puls und/oder pTx-Puls. Vorzugsweise wird in S50 der Anregungspuls hinsichtlich einer Homogenität einer durch den Anregungspuls erzeugbaren Magnetfeldverteilung und/oder einer SAR-Exposition durch den Anregungspuls optimiert. Vorzugsweise ist der Anregungspuls räumlich selektiv, insbesondere schichtselektiv. Insbesondere wird durch den Anregungspuls ein bestimmter Bereich, beispielsweise eine bestimmte Schicht, innerhalb des Patientenaufnahmebereichs 14 bzw. des Patienten 15 angeregt.

In S60 wird zumindest ein Refokussierungspulses ermittelt, der die in S30 vorgegebene Refokussierungspulsform aufweist. Vorzugsweise umfasst der zumindest eine Refokussierungspuls mehrere Refokussierungspulse, d.h. es werden in S60 mehrere Refokussierungspulse erzeugt; die mehreren Refokussierungspulse werden vorzugsweise in der Magnetresonanzsequenz nacheinander appliziert; vorteilhafterweise kann somit ein Echozug erzeugt werden. Vorzugsweise ist Magnetresonanzsequenz eine RARE-Sequenz (Rapide Acquisition with Relaxation Enhancement), insbesondere eine TSE-Sequenz (Turbo Spin Echo) und/oder FSE-Sequenz (Fast Spin Echo).

Vorzugsweise ist der zumindest eine Refokussierungspuls räumlich selektiv, insbesondere schichtselektiv. Insbesondere wird durch den Refokussierungspuls ein bestimmter Bereich, beispielsweise eine bestimmte Schicht, innerhalb des Patientenaufnahmebereichs 14 bzw. des Patienten 15 angeregt. Vorzugsweise ist die in S30 vorgegebene Refokussierungspulsform dahingehend optimiert, die gewünschte räumliche Selektion zu bewirken. Beispielsweise weist der Refokussierungspulsform eine Form eines, insbesondere adaptierten, Sinc-Pulses und/oder eines SLR-Pulses auf. Diese Pulse eigenen sich besonders gut, um ein räumlich präzise definiertes Schichtprofil anzuregen.

In S70 wird die Magnetresonanzsequenz mit dem Anregungspuls und dem zumindest einen Refokussierungspuls bereitgestellt. Die Magnetresonanzsequenz kann insbesondere für mehrere Sendekanäle jeweils einen Anregungspuls und zumindest einen Refokussierungspuls aufweisen.

S20, S30, S40, S50, S60 und S70 können beispielsweise mittels der Systemsteuereinheit 22 der Magnetresonanzvorrichtung 10 ausgeführt werden. In S80 wird mit der Magnetresonanzvorrichtung 10 eine Magnetresonanzmessung gemäß der in S70 bereitgestellten Magnetresonanzsequenz durchgeführt.

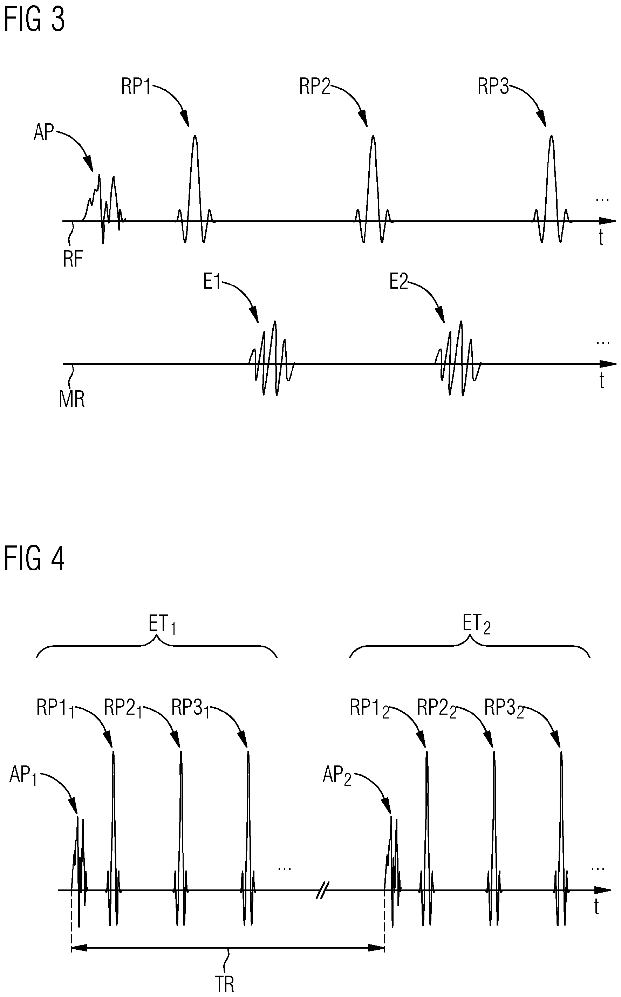

Anhand der Sequenzdiagramme der Fig. 3, 4 und 5 sollen verschiedene mögliche Aspekte des Verfahrens zum Bereitstellen der Magnetresonanzsequenz näher erläutert werden. Die Elemente der Magnetresonanzsequenzen sind dabei auf einem Zeitstrahl t angeordnet.

Der in S50 ermittelte Anregungspuls ist vorzugsweise ein schichtselektiver, dynamischer pTx-Anregungspuls, der noch freier parametrisiert ist als die herkömmlichen Pulse. Beispielhaft ist ein solcher Anregungspuls AP in Fig. 3 dargestellt. Zeitlich darauffolgend sind drei Refokussierungspulse RP1, RP2 und RP3 dargestellt, die eine vorgegebene Pulsform aufweisen, die in diesem Beispiel die Form einer Sinc-Funktion hat. Eine weitere bevorzugte Pulsform wäre beispielsweise die eines SLT-Pulses. Die Refokussierungspulse RP1, RP2, RP3 werden mit dem Anregungspuls AP zu einer Magnetresonanzsequenz bzw. Pulssequenz kombiniert. Die Magnetresonanzsequenz kann auch noch mehr Refokussierungspulse aufweisen.

In den Fig. 3, 4 und 5 sind lediglich die, insbesondere reellwertigen, Momentanwerte der Pulse bzw. Pulsprofile dargestellt. Es ist ferner denkbar, dass die Pulse bzw. Pulsprofile eine, insbesondere von Null abweichende, Phase aufweisen. Die Pulse bzw. Pulsprofile können dann insbesondere durch eine komplexwertige Funktion beschrieben werden.

Die Refokussierungspulse RP1, RP2 und RP3 bewirken eine Umkehrung der Phase der durch den Anregungspuls AP angeregten Kernspins. Dadurch werden Spinechos E1, E2, ... generiert, die als Magnetresonanzsignale von der Hochfrequenzantenneneinheit 20 aufgenommen werden können.

Vorzugsweise sind die Refokussierungspulse RP1, RP2 und RP3 "einfach geshimmt" gemäß einer "direkten Signaloptimierung"; bei einer "direkten Signaloptimierung" wird für jeden Puls in der Pulssequenz ein B1-Shim bzw. ein B1-Feld errechnet und angewendet. So kann die Homogenität verbessert werden; allerdings ist die mögliche Verbesserung dadurch limitiert, dass ein einzelner Puls auch nur hinsichtlich eines einzelnen B1-Feldes optimiert wird. Die Homogenität, insbesondere der erzeugten Flipwinkel, insgesamt ist dadurch üblicherweise nicht auf dem hohen Niveau von dynamischen pTx-Pulsen.

Der Anregungspuls AP ist vorzugsweise ein freier, dynamischer pTx-Puls, der aus beliebigen HF-Pulsformen und auch insbesondere Gradientenpulsformen bestehen kann. (Hingegen sind die Refokussierungspulse RP1, RP2 und RP3 formgebunden und bestehen beispielsweise aus einem oder mehreren Sinc-förmigen HF-Subpulsen, die also eine vorgegebene Pulsform aufweisen, gepaart mit Schichtselektionsgradienten.) Ein freier, dynamischer pTx-Puls, der aus beliebigen HF-Pulsformen besteht, ermöglicht eine mehr verschiedene B1- und Gradientenfelder (Freiheitsgrade für die Optimierung) und ist insbesondere nicht an die Sinc-Formen gebunden. Damit kann vorteilhafterweise eine homogene Anregung bei wenig SAR innerhalb der Schicht erzeugt werden. Aufgrund etwaiger Ungenauigkeiten in der Kalibrierung (beispielsweise hinsichtlich von HF-Leistungsverstärkern der Hochfrequenzantenneneinheit 20, der B1-Felder und/oder der B0- Felder), werden solche Pulse allerdings anfällig für schlechtere Schichtprofile.

Vorteilhafterweise wird durch den Anregungspuls AP innerhalb der anzuregenden Schicht allerdings ein homogener Flipwinkel und eine entsprechende Phase der Kernspins dabei so erzeugt, dass darauffolgende Sinc-förmige Refokussierungspulse RP1, RP2 und RP3 mit einem bestimmten B1-Shim dasselbe Phasenprofil erzeugen können (+90° = Differenz zwischen der Phase des Anregungspulses und des Refokussierungspulses). Dadurch kann es ausreichend sein, eine direkte Signaloptimierung nur für die Refokussierungspulse durchzuführen. Die bessere Homogenität, die am Anfang durch den Anregungspuls AP erzeugt wird, bleibt dabei über mehrere Echos hinweg erhalten.

Die Refokussierungspulse EP1, EP2, EP3 "schneiden" vorteilhafterweise ein genaues Schichtprofil aus dem eventuell ungenauen Schichtprofil des Anregungspulses AP heraus und stellen so die gewünschte Schichtselektion sicher. Dadurch, dass sie selbst kurz und keine dynamischen pTx-Pulse sind, ermöglichen sie vorteilhafterweise eine Robustheit gegenüber B0-Inhomogenitäten, Bewegung und verringern T2-Blurring. Eine Kombination der beschriebenen Refokussierungspulse EP1, EP2, EP3 mit dem beschriebenen Anregungspuls AP führt vorteilhafterweise zu einer deutlichen Verbesserung der Homogenität und weniger SAR-Exposition im Vergleich zur reinen direkten Signaloptimierung.

In Fig. 4 wird gezeigt, dass eine Magnetresonanzsequenz auch mehrere Echozüge ET₁, ET₂ umfassen kann, die jeweils einen Anregungspuls AP₁ bzw. AP₂ und mehrere Refokussierungspulse RP1₁, RP2₁, RP3₁ bzw. EP1₂, EP2₂, EP3₂ umfasst. Die Anregungspulse AP₁ und AP₂ weisen einen zeitlichen Abstand TR (Repetitionszeit, engl. repetition time) auf.

Man kann AP₁, RP1₁, RP2₁, RP3₁ als eine erste Magnetresonanzsequenz und AP₂, RP1₂, RP2₂, RP3₂ als eine zweite Magnetresonanzsequenz auffassen, die Teil einer Gesamtmagnetresonanzsequenz sind. Damit können die erste Magnetresonanzsequenz und die zweite Magnetresonanzsequenz (und etwaige weitere Magnetresonanzsequenzen) gemäß einem in Fig. 2 dargestellten Verfahren bereitgestellt werden.

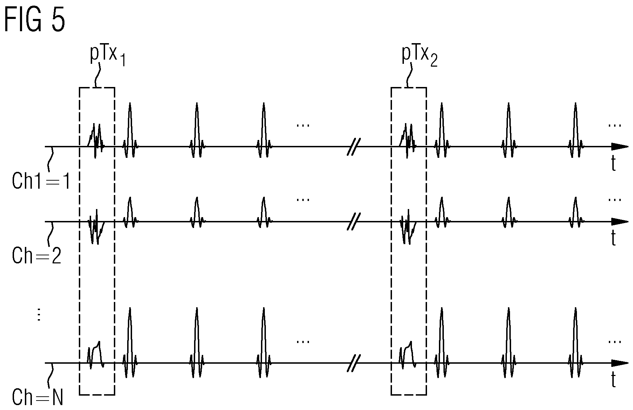

In Fig. 5 sind mehrere Echozüge mehrerer Sendekanäle der Magnetresonanzvorrichtung 10 dargestellt. Zu Beginn eines Echozugs werden parallel auf jedem der Sendekanäle Ch = 1, 2, ... N jeweils ein Anregungspuls angewendet, die zusammen als pTx-Puls pTx₁, pTx₂ aufgefasst werden können. Insbesondere können der Anregungspuls des Kanals Ch = 1 als erster Teilpuls, der Anregungspuls des Kanals Ch = 2 als zweiter Teilpuls, ..., und der Anregungspuls des Kanals Ch = N als Nter Teilpuls eines pTx-Pulses pTx₁, pTx₂ aufgefasst werden. Dabei kann jeder der N Teilpulse eine andere Pulsform aufweisen. Die Pulsform ist insbesondere abhängig von dem in S20 bereitgestellten Magnetfeldverteilungsdatensatz. Die (pro Sendekanal skalierte) Pulsform der auf die Anregungspulse folgenden Refokussierungspulse ist dagegen immer dieselbe, weil fest vorgegeben.

Es wird abschließend noch einmal darauf hingewiesen, dass es sich bei den vorhergehend detailliert beschriebenen Verfahren sowie bei der dargestellten Magnetresonanzvorrichtung lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden TeilKomponenten bestehen, die gegebenenfalls auch räumlich verteilt sein können. Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zum Bereitstellen einer Magnetresonanzsequenz, insbesondere einer Spin-Echo-Sequenz,
- Bereitstellen eines Magnetfeldverteilungsdatensatzes,
- Vorgeben einer Refokussierungspulsform,
- Ermitteln eines, insbesondere parallelen, Anregungspulses, der eine Anregungspulsform aufweist,
wobei die Anregungspulsform in Abhängigkeit von dem Magnetfeldverteilungsdatensatz ermittelt wird,
- Ermitteln zumindest eines, insbesondere parallelen, Refokussierungspulses, der die vorgegebene Refokussierungspulsform aufweist,
- Bereitstellen der Magnetresonanzsequenz mit dem Anregungspuls und dem zumindest einen Refokussierungspuls.

2. Verfahren nach Anspruch 1,
wobei der Anregungspuls ein dynamischer Puls ist.

3. Verfahren nach einem oder vorangehenden Ansprüche,
wobei die Magnetresonanzsequenz für mehrere Sendekanäle jeweils einen Anregungspuls und zumindest einen Refokussierungspuls umfasst.

4. Verfahren nach einem oder vorangehenden Ansprüche,
wobei der Magnetfeldverteilungsdatensatz eine B1-Feldkarte umfasst.

5. Verfahren nach einem oder vorangehenden Ansprüche,
wobei die Ermittlung der Anregungspulsform eine Optimierung einer Homogenität einer durch den Anregungspuls erzeugbaren Flipwinkelverteilung und/oder eine Optimierung einer SAR-Exposition durch den Anregungspuls umfasst.

6. Verfahren nach einem oder vorangehenden Ansprüche,
wobei die Ermittlung der Anregungspulsform eine Optimierung einer Homogenität der Flipwinkelverteilung in nur einem Teilbereich einer Schicht umfasst.

7. Verfahren nach einem der vorangehenden Ansprüche,
wobei der Anregungspuls und/oder der zumindest eine Refokussierungspulses räumlich selektiv, insbesondere schichtselektiv, sind.

8. Verfahren nach einem der vorangehenden Ansprüche,
wobei Refokussierungspulsform eine Form eines, insbesondere adaptierten, Sinc-Pulses und/oder eines SLR-Pulses und/oder eines Rechteck-Pulses aufweist.

9. Verfahren nach einem der vorangehenden Ansprüche,
wobei die Ermittlung des zumindest eines Refokussierungspulses anhand in Abhängigkeit von dem Magnetfeldverteilungsdatensatz erfolgt.

10. Verfahren nach einem der vorangehenden Ansprüche,
wobei das Ermitteln des zumindest eines Refokussierungspulses eine Ermittlung eines Skalierungsfaktors in Abhängigkeit von dem Magnetfeldverteilungsdatensatz umfasst,
wobei der Refokussierungspuls anhand der Refokussierungspulsform und dem Skalierungsfaktor ermittelt wird.

11. Verfahren nach Anspruch 10,
wobei die Magnetresonanzsequenz ein Senden des Anregungspuls und des zumindest einen Refokussierungspulses mittels mehrerer Sendekanäle vorsieht,
wobei für jeden Sendekanal ein kanalspezifischer Skalierungsfaktor ermittelt wird.

12. Verfahren nach einem oder vorangehenden Ansprüche,
wobei der zumindest eine Refokussierungspuls mehrere Refokussierungspulse umfasst.

13. Verfahren nach einem der vorangehenden Ansprüche,
wobei die Magnetresonanzsequenz eine RARE-Sequenz (Rapide Acquisition with Relaxation Enhancement), insbesondere eine TSE-Sequenz (Turbo Spin Echo) und/oder FSE-Sequenz (Fast Spin Echo), ist.

14. Magnetresonanzvorrichtung, die ausgebildet ist, ein Verfahren nach einem der vorangehenden Ansprüche auszuführen.

15. Computerprogrammprodukt, welches ein Programm umfasst und direkt in einen Speicher einer programmierbaren Systemsteuereinheit einer Magnetresonanzvorrichtung ladbar ist, mit Programmmitteln, um ein Verfahren nach einem der Ansprüche 1 bis 13 auszuführen, wenn das Programm in der Systemsteuereinheit der Magnetresonanzvorrichtung ausgeführt wird.
